(19) Europäisches Patentamt
European Patent Office
Office européen des brevets

(11) Numéro de publication: **0 093 627**
**B1**

(12) # FASCICULE DE BREVET EUROPEEN

(45) Date de publication du fascicule du brevet: **10.07.85**

(51) Int. Cl.⁴: **C 07 C 51/41**

(21) Numéro de dépôt: **83400626.4**

(22) Date de dépôt: **25.03.83**

(54) **Procédé pour la préparation de carboxylates cériques.**

(30) Priorité: **12.04.82 US 367310**

(43) Date de publication de la demande:
**09.11.83 Bulletin 83/45**

(45) Mention de la délivrance du brevet:
**10.07.85 Bulletin 85/28**

(84) Etats contractants désignés:
**AT BE CH DE FR GB IT LI NL SE**

(56) Documents cités:
**CH - A - 505 758**

**Chemical Abstracts vol. 86, no. 12, 21 mars 1977, Columbus, Ohio, USA B.L. KALSOTRA et al. "Cerium (IV)carboxylates", page 723, colonne 1, abstract no. 83016j**

(73) Titulaire: **RHONE-POULENC SPECIALITES CHIMIQUES, "Les Miroirs" 18, Avenue d'Alsace, F-92400 Courbevoie (FR)**

(72) Inventeur: **Gradeff, Peter S., P.O. Box 278, Pottersville New Jersey (US)**
Inventeur: **Charte, Vincent J., 881 Jamestown Road, East Windsor New Jersey 08520 (US)**

(74) Mandataire: **Savina, Jacques et al, RHONE-POULENC RECHERCHES Service Brevets Chimie et Polymères 25, quai Paul Doumer, F-92408 Courbevoie Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

**Description**

Des savons préparés à partir de métaux lourds tels que le cobalt, le plomb, le vanadium, le zirconium, le nickel et autres, sous la forme de naphténates, sont largement utilisés à titre d'agents accélérateurs et dessicateurs pour rendre plus rapide le séchage et le durcissement des vernis et peintures à base d'huile. On les utilise également en tant qu'additifs on adjuvants dans des compositions pour matières plastiques, agents ignifugeants, silicones et combustibles.

Il est également connu que les savons à base de cérium ont une action siccative mais du fait de leur prix, leur utilisation restait, jusqu'à présent, peu développée. Récemment, ces savons ont été proposés à titre d'adjuvants ou d'additifs dans des combustibles, dans des compositions non-inflammables (ignifugeantes), des matières plastiques et des silicones et ils s'avèrent donc offrir d'autres applications potentielles. Les savons au cérium qui sont décrits dans la littérature sont des savons céreux. Pour autant que la demanderesse en ait connaissance, il n'existe dans cette littérature qu'une seule description de savons cériques ou de leur préparation, et c'est celle faite par B. L. Kalsotra et des collaborateurs, dans »Transition Metal Chemistry, 1, 159—181 (1976)«, qui indique qu'une bonne partie des travaux a été effectuée sur des carboxylates céreux »mais qu'il n'y a par contre aucune référence dans la littérature sur la préparation de carboxylates cériques«. L'article a pour objet la préparation et la caractérisation de carboxylates cériques par la réaction de $H_2CeCl_6$ avec (respectivement) $HCO_2H$, un mélange de $(MeCO)_2O$,

$EtCO_2H$, n-$PrCO_2H$ ou 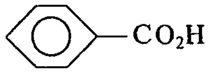 selon l'équation:

$$H_2CeCl_6 + RCOOH \longrightarrow Ce(OOCR)Cl_3 \times H_2O + 3\,HCl$$

Les carboxylates qui sont préparés conformément au procédé indiqué, contiennent trois atomes de chlore. Des tentatives faites par Kalsotra et ses collaborateurs pour préparer des carboxylates cériques par la réaction de l'hexachlorure dipyridino-cérique $(C_6H_5N)_2CeCl_6$ avec des sels de sodium d'acides gras, d'une façon qui est analogue à la péparation des composés du type cyclopentadiényl-cérique et indényl-cérique, ont échoué.

Toutefois, il existe sur le marché des savons de cérium qui sont en fait un mélange de carboxylates céreux et cériques: ce sont des naphténates de cérium qui contiennent de 30 à 50% de naphténate cérique. Le procédé pour leur préparation n'a pas été divulgué; ils sont préparés ou fabriqués en France.

Le procédé que l'on applique lors de la préparation des savons de métaux lourds dépend de la réactivité de métal particulier ou de ses dérivés. Les procédés que l'on applique le plus communément sont:

(a) l'échange anionique par déplacement d'un anion inorganique par un anion carboxylate, que l'on effectue en ajoutant une base à une solution aqueuse convenablement agitée d'un sel métallique (sel inorganique) en présence d'une solution de l'acide carboxylique désiré dans un solvant approprié qui n'est pas miscible avec l'eau;

(b) la précipitation du savon métallique à partir de solutions aqueuses des sels métalliques avec des savons alcalins;

(c) la fusion d'oxydes, d'hydroxydes ou de sels de métaux avec des acides ou des esters organiques, et

(d) la réaction directe des métaux à l'état finement divisé dans des acides organiques chauffés.

En ce qui concerne la façon de procéder, les techniques (a) et (b) sont les plus faciles à mettre en œuvre et elles sont également celles qui s'avèrent être les plus économiques. Elles sont applicables au cérium tout aussi bien qu'à de nombreux autres métaux. On peut se servir d'un sel céreux courant quelconque, soluble comme le nitrate céreux ou le chlorure céreux, dans le procédé (a) pour la préparation des savons céreux. Théoriquement, par analogie avex des savons céreux, il est possible de se servir de nitrate céri-ammoniacal ou de sulfate cérique pour préparer des savons cériques. Il n'y a pas de référence dans la littérature qui précise que ceci ait jamais été effectué. Le problème en est que le nitrate céri-ammoniacal ou le sulfate cérique qui sont les seuls sels disponibles sur le marché, qui soient solubles dans l'eau, ont un prix élevé. Ceci représente peut-être l'une des raisons pour lesquelles on ne trouve décrits dans la littérature que des savons céreux et non des savons cériques.

Lorsque l'on désire avoir des savons métalliques solides, c'est le procédé (b) qui est le plus approprié et qui est applicable au cérium. Il est possible de se servir d'un sel quelconque de cérium qui est soluble, tel que le nitrate ou le chlorure céreux, pour préparer des savons céreux sous une forme solide. Si, d'une manière théorique, il est possible d'utiliser des sels cériques qui sont solubles dans l'eau comme l'est le nitrate céri-ammoniacal ou le sulfate cérique, et dont on se sert peut-être lors de la préparation des naphténates mixtes céreux/cériques qui sont disponibles, des savons cériques se présentent sous forme de substances soit liquides soit cireuses et qui sont difficiles à isoleur du mélange réactionnel.

De plus, il est également onéreux de préparer des savons cériques d'après le procédé (b), parce que les seuls sels inorganiques cériques solubles sont le nitrate céri-ammoniacal et le sulfate cérique qui sont coûteux.

L'hydroxyde cérique présente une réactivité faible et une médiocre solubilité et il ne convient pas aux procédés (a) ni (b).

Conformément à la présente invention, l'utilisation du nitrate céri-ammoniacal ou du sulfate cérique qui sont coûteux, est évitée par la mise en œuvre du savon céreux correspondant et en oxydant le savon céreux en savon cérique à l'aide d'une solution aqueuse de peroxyde d'hydrogène.

Le problème que pose la manipulation et la récupération du savon cérique dans le mélange réactionnel, se trouve ainsi éliminé car la réaction d'oxydation par le peroxyde d'hydrogène est conduite dans un système à deux phases qui comprend une phase aqueuse de péroxyde d'hydrogène dont la valeur du pH est d'au moins 6 et une phase organique qui est constituée par une solution de carboxylate céreux dans un solvant hydrocarboné non miscible avec l'eau, cette réaction transforme l'ion céreux en ion cérique et forme une solution de carboxylate cérique dans le solvant hydrocarboné. Ensuite, lorsque la réaction est achevée, on chauffe le mélange réactionnel à une température à laquelle sont décomposés d'éventuels complexes cériques du peroxyde d'hydrogène, et l'on sépare alors la phase organique de la phase aqueuse du mélange réactionnel. Le carboxylate cérique obtenu est normalement en solution, telle qu'on l'utilise et il peut être récupéré à partir de la phase organique, par les méthodes bien connues de l'homme de l'art comme, par exemple: la distillation du solvant à basse température et sous une faible pression.

Théoriquement, la réaction est à même de transformer quantitativement le carboxylate céreux en carboxylate cérique. Il n'est cependant pas nécessaire, pour la plupart des besoins industriels, d'effectuer une transformation complète. Le rapport entre $Ce^{4+}$ produit et le total du cérium présent peut varier, si on le désire, entre 1% et aller jusqu'à 100%.

Il est permis d'apprécier l'utilité de 1% seulement de $Ce^{4+}$ dans des solutions de carboxylate de $Ce^{3+}$ en s'appuyant sur la réduction inattendue de la viscosité du mélange des carboxylates en solution ce qui, sans cela, pourrait soulever des problèmes. Le carboxylate céreux comme le carboxylate cérique sont en général tous deux chimiquement équivalents à titre de source de cérium lors de réactions dans lesquelles le cérium est souhaité, comme dans le cas des agents de dessication et accélérateurs. De plus, les solutions de carboxylate cérique dans des solvants organiques ont une viscosité bien plus faible que les solutions organiques des carboxylates céreux, à des concentrations similaires du cérium en milieu organique, et cette diminution de la viscosité est déjà marquée lorsque le cérium n'est qu'à 1% sous forme cérique. Par conséquent, le procédé selon l'invention se révèle particulièrement intéressant dans la plupart des cas et notamment pour transformer une quantité aussi faible que 1% de cérium céreux en cérium cérique. Malgré cela, il est généralement souhaitable de transformer entre 30 et 60% et de préférence de 50 à 95% de cérium céreux en cérium cérique, pour des applications dans lesquelles on recherche une teneur plus élevée en $Ce^{4+}$.

Selon l'invention, on met en œuvre du carboxylate céreux comme matière première en solution dans un solvant hydrocarboné qui n'est pas miscible avex l'eau, et la solution aqueuse de peroxyde d'hydrogène dont la valeur du pH est d'au moins 6, pour former un système réactionnel à deux phases. Selon une variante du procédé de l'invention, on peut aussi former in situ le carboxylate céreux à partir d'un composé céreux et d'un composé du type carboxylate, par exemple à partir d'un sel céreux inorganique comme le nitrate céreux ou le sulfate céreux, et de l'acide carboylique avec un alcali, ou d'un sel de l'acide carboxylique. Le carboxylate céreux se forme in situ, en tant que produit intermédiaire, et à l'issue du procédé de l'invention, le produit obtenu est un carboxylate cérique en solution dans le solvant.

Lorsque l'on utilise un carboxylate céreux comme matière première, le procédé de l'invention est présentement décrit comme Procédé I, et si l'on se sert d'un composé céreux et d'un composé du type carboxylate comme matière de départ, on se réfère alors au procédé décrit comme Procédé II. Ces deux procédés, bien qu'ils soient similaires en principe, sont assez différents quant à leur mise en œuvre, pour cette raison ils sont décrits séparément ci-après.

Le Procédé I, par le fait qu'il implique la mise en œuvre d'un carboxylate céreux comme produit de départ, peut aussi comprendre la préparation dudit carboxylate céreux à partir d'un sel céreux inorganique et d'un acide carboxylique, et dans ce cas on peut se servir directement du mélange réactionnel lors de l'oxydation par le peroxyde d'hydrogène, sans séparation de la solution de carboxylate céreux de la couche aqueuse. Ceci est une voie des plus économiques pour la mise en œuvre du procédé de l'invention, c'est celle que l'on préfère par conséquent.

Alors que la voie la plus pratique et la plus économique de production des savons cériques consiste à introduire une étape d'oxydation dans le processus de fabrication des savons céreux, on peut également appliquer l'oxydation à la solution d'un savon céreux quelconque dans un solvant hydrocarboné, par l'addition d'une solution aqueuse de peroxyde d'hydrogène. Par example, il est possible de mélanger une solution quelconque du commerce d'un carboxylate céreux avec une solution aqueuse de peroxyde d'hydrogène, ce qui amène la réaction d'oxydation à se produire. On peut aussi ajouter un excès d'acide carboxylique, de préférence sous la forme d'un sel d'ammonium ou d'un sel de métal alcalin.

Procédé I

Il est possible de mettre en œuvre le procédé (a) ci-dessus lors de la synthèse de carboxylate céreux. On ajoute une base, telle qu'un hydroxyde d'un métal alcalin, par exemple de sodium ou de potassium, ou de l'hydroxyde d'ammonium, à un système à deux phases qui contient un solvant non miscible avec l'eau et une solution aqueuse convenablement agitée d'un sel céreux soluble tel que le nitrate céreux, le chlorure céreux ou le sulfate céreux et l'acide carboxylique correspondant. Le mélange est effectué selon les proportions stoechiométriques, mais de préférence en utilisant un excès de l'acide. Le carboxylate céreux que l'on obtient de cette façon est ensuite extrait du mélange réactionnel aqueux, à l'aide d'un solvant hydrocarboné qui n'est pas miscible avec l'eau, et dans lequel il est soluble; ce solvant est avantageusement présent au moment de l'addition de la base, ce qui conduit ainsi à la présence d'un système à deux phases dès cette étape, on bien il est introduit après la fin de la précipitation du carboxylate céreux à partir de la phase aqueuse. On peut ensuite séparer la phase organique du carboxylate céreux dissous dans le solvant hydrocarboné de la phase aqueuse, mais ceci n'est pas nécessaire.

Stoechiométriquement, il est requis de disposer de trois moles d'acide carboxylique par mole de $Ce^{3+}$ sous forme de sel, pour obtenir le carboxylate de $Ce^{4+}$. Théoriquement, une mole supplémentaire d'acide est nécessaire si l'on veut que la totalité du $Ce^{3+}$ soit transformé en $Ce^{4+}$ sous la forme du carboxylate cérique. Le fait est que l'oxydation a bien lieu avec une quantité moindre que la valeur théorique d'acide, ceci est une indication d'après laquelle le carboxylate de $Ce^{4+}$ produit par ce procédé contient d'autres fonctions que les fonctions hydroxyles.

La base mise en œuvre dans le procédé (a) pour la préparation de savons céreux peut être de l'hydroxyde de sodium ou de potassium, leurs carbonates ou leurs bicarbonates ou l'hydroxyde d'ammonium. La quantité de base est importante; il est requis d'avoir au moins la quantité stoechiométrique de cérium de façon à pouvoir transformer la totalité du cérium en carboxylate de cérium soluble dans le solvant et d'ajuster la valeur du pH à 6 au moins et de préférence à une valeur supérieure à 7, avant l'oxydation à l'aide du peroxyde d'hydrogène.

On ajoute le peroxyde d'hydrogène sous la forme d'une solution aqueuse peu après que soit achevée l'addition de la base et avant la séparation des deux phases. De préférence, le pH du mélange réactionnel, avant l'addition du $H_2O_2$ est supérieur à 6. Bien qu'il soit possible de modifier l'ordre de la combinaison des agents réactifs, pour parvenir aux meilleurs résultats, l'addition de $H_2O_2$ doit être effectuée en dernier lieu.

On conserve le mélange réactionnnel vigoureusement agité alors que l'on effectue l'addition de la solution aqueuse de peroxyde d'hydrogène. La réaction débute à la température ambiante et il se forme rapidement, dans le mélange réactionnel, une coloration brune foncée rougeâtre. On sait que le peroxyde d'hydrogène forme des complexes colorés qui sont solubles dans l'eau, avec des ions inorganiques et probablement c'est la même chose qui se produit dans ce cas, avec l'ion cérique. Dans le procédé selon la présente invention le système de carboxylate de cérium et du peroxyde d'hydrogène forme une solution du solvant organique colorée foncée, en raison de ce complexe qui, par chauffage, se décompose avec dégagement du $H_2O_2$. Par conséquent, après une période brève au cours de laquelle une certaine quantité de peroxyde d'hydrogène oxyde le $Ce^{3+}$ en $Ce^{4+}$ et que le reste se trouve immobilisé par la formation dudit complexe, on porte la temérature du mélange réactionnel jusqu'à environ 60 à 75° C, pour détruire les complexes cériques de $H_2O_2$, à la suite de quoi la coloration s'éclaircit pour donner une coloration orangée claire permanente qui est l'indicateur de la décomposition desdits complexes.

La quantité de peroxyde d'hydrogène que l'on peut ajouter varie considérablement en fonction de la proportion souhaitée de la transformation du cérium de l'état céreux à l'état cérique, de 5% à plus de 90%. Une conversion à 100% est difficile à obtenir par le fait qu'il semble se produire un certain degré de réduction du $Ce^{4+}$ en $Ce^{3+}$ qui, dans certains cas, est fonction ou dépend de l'ion carboxylate qui serait responsable d'une limitation de la teneur maximale en $Ce^{4+}$. Il est possible aussi de se servir de petites quantités de $H_2O_2$ ce qui a pour effet d'avoir une moindre transformation de cérium de l'état céreux à l'état cérique. Une faible transformation comprise entre 1 et 5% pourrait être souhaitable pour certaines application. Le degré de la conversion dépend aussi de la nature de l'acide carboxylique et des impuretés qui sont présentes. A titre d'exemple, le degré de la transformation de l'état céruex en cérique est plus important dans le cas de l'acide néo-décanoïque que dans le cas de l'acide naphténique.

Pour parvenir à une transformation élevée du céreux en cérique, il peut être nécessaire d'effectuer deux ou plusieurs additions de peroxyde d'hydrogène, suivies à chaque fois par une séquence de chauffage-refroidissement, pour décomposer les complexes $H_2O_2$ cériques, ceci en fonction de l'anion carboxylate. Dans le cas de certains carboxylates, une seule addition de $H_2O_2$ est suffisante pour élever la teneur de cérium à l'état cérique à plus de 90%. Avec d'autres carboxylates, pour pouvoir atteindre 90%, il est nécessaire de procéder à deux ou trois additions de $H_2O_2$, tel par exemple qu'avec le naphténate cérique. Un explication possible de ce fait réside en ce que le mélange qui est vendu pour être de l'acide naphténique est déjà oxydable par l'ion cérique et cette réaction secondaire consomme l'ion cérique en le transformant en ion céreux, ce qui diminue la teneur en ion cérique.

Comme il a été précédemment indiqué, la quantité d'acide carboxylique doit être supérieure à celle qui, stoechiométriquement, est requise pour avoir un savon céreux, à savoir de 3 moles par atome-gramme de cérium. Le savon cérique correspondant, préparé conformément à la présente invention, peut contenir un certain nombre de radicaux OH ou d'autres liaisons, ce qui est acceptable pour de nombreuses applications. Assez souvent, des solutions de savons céreux contiennent un excès de l'acide carboxylique correspondant. Il est préférable de se servir d'environ 4 moles d'acide carboxylique, de façon à avoir la quantité qui est stoechiométriquement requise pour le savon cérique que l'on doit préparer. On peut utiliser, si on le désire, plus de 4 Moles d'acide.

Procédé II

Conformément à ce procédé, on oxyde l'ion céreux dans le mélange réactionnel par l'addition d'une solution aqueuse de peroxyde d'hydrogène:

(1) à une solution aqueuse d'un carboxylate alcalin et avant l'addition de la solution aqueuse de sel céreux, ou bien
(2) à une solution aqueuse d'un sel céreux inorganique soit (a) avant le mélange avec une solution d'un carboxylate alcalin, soit (b) à une vitesse contrôlée au cours du mélange avec la solution du carboxylate alcalin. La présence d'un solvant organique au cours de la réaction est préférable, pour permettre l'extraction du savon cérique au fur et à mesure de sa formation.

La quantité de peroxyde d'hydrogène mise en œuvre n'est pas critique et elle peut varier d'une quantité inférieure à la valeur stoechiométrique requise jusqu'à un fort excès. Normalement, on préfère avoir un excès à cause de la tendance de l'ion cérique à former des complexes avec le $H_2O_2$. Après le mélange du carboxylate alcalin et du sel céreux on peut ajouter une quantité supplémentaire de peroxyde d'hydrogène, pour augmenter la teneur en ion cérique, si on le désire. Dans certains cas, il peut être nécessaire de chauffer la solution jusqu'à une température de 65 à 70° C, pour détruire le complexe cérique de $H_2O_2$ coloré, avant d'ajouter une nouvelle quantité de $H_2O_2$.

On effectue de préférence l'addition du $H_2O_2$ à la température ambiante, mais la température peut aller jusqu'à 70° C, avec l'inconvénient selon lequel la réaction est moins efficace du fait d'une perte en $H_2O_2$. A la fin de cette réaction, on chauffe le mélange réactionnel de 65 à 75° C, afin de décomposer le complexe cérique de $H_2O_2$ et détruire tout excès de $H_2O_2$.

Selon les deux procédés, il est possible d'améliorer le rendement par l'extraction de la phase aqueuse à l'aide d'un solvant organique et par la combinaison avec la phase organique, lorsque le carboxylate cérique est difficilement soluble dans l'eau.

On peut appliquer le procédé selon la présente invention pour la transformation d'un savon céreux quelconque que l'on peut dissoudre dans un solvant hydrocarboné qui n'est pas miscible avec l'eau en quantité suffisante pour permettre à la réaction de se produire. Le procédé présente un intérêt industriel particulier pour la préparation de naphténate cérique, de 2-éthyl-hexoate cérique ou de néo-décanoate cérique, mais il va de soi que l'on peut tout aussi bien l'utiliser à la préparation d'un carboxylate cérique quelconque que l'on pourrait souhaiter, d'un acide carboxylique quelconque, aliphatique ou cycloaliphatique, saturé ou non saturé, ou des mélanges de ces acides qui présentent entre 7 et 18 atomes de carbone, en partant du carboxylate céreux correspondant.

Des exemples d'acides carboxyliques qui fournissent l'anion carboxylate comprennent les acides caprique, 2-éthyl-hexoïque, caprylique, laurique, myristique, stéarique, palmitique, oléïque, linoléïque, ricinoléïque, naphténique, méthyl-cyclohexanoïque, méthyl-cyclohexanoïque, cyclo-heptanoïque et les mélanges des acides gras qui sont dérivés des huiles et graisses naturelles, comme de ceux d'huile de noix de coco, les acides gras du suif, les acides gras du lard, les acides gras de l'huile de maïs, les acides gras de l'huile de graines de lin, les acides gras de l'huile d'abrasin, les acides gras de l'huile de graines de colza, les acides grad de l'huile de graines de coton, les acides gras des huiles de poissons, les acides grad de l'huile des graines de soja et les acides gras de l'huile de graines de carthame.

Si le carboxylate céreux n'est pas disponible, on combine le procédé selon la présente invention avec sa préparation, à titre de première étape, en se servant d'un sel céreux, soluble dans l'eau, comme le nitrate ammonio-céreux, le nitrate céreux, le sulfate céreux ou l'acétate céreux avec l'acide carboxylique libre, selon la quantité stoechiométriquement équivalente en tant que matières de départ.

Il est possible de se servir d'un solvant hydrocarboné liquide quelconque, qui ne soit pas miscible avec l'eau, dans lequel peut être dissout le savon céreux pour former la phase organique du mélange réactionnel. Ces solvants sont notamment choisis parmi des solvants d'hydrocarbures aliphatiques, cycloaliphatiques et aromatiques qui présentent entre six et quatorze atomes de carbone, comme le sont par exemple les éthers de pétrole, qui sont composés d'hydrocarbures paraffiniques, d'hydrocarbures cycloaliphatiques et leurs mélanges, l'hexane, l'heptane, l'octane, le nonane, le décane, le dodécane, le tétradécane, le cyclohexane, le cycloheptane, le cyclopentane, le cyclooctane, le cyclo-

hexène, le cycloheptène, le cyclooctène, le benzène, le toluène, le p-cymène, le pseudo-cumène, les xylènes, le mésitylène, l'éthylbenzène, le 1,2,3-triméthylbenzène, le tétraméthylbenzène, le propylbenxène, l'isopropylbenzène, les dipropylbenzènes et les di-isopropylbenzènes.

Les exemples qui vont suivre illustrent la présente invention.

Exemple 1

Dans un ballon à quatre tubulures, à fond rond, de trois litres muni d'un agitateur mécanique à grande vitesse, d'un thermomètre, d'un dispositif de refroidissement et d'un entonnoir d'addition, on place 146,8 g d'une solution aqueuse de nitrate céreux (23,86% de céreux), 0,250 mole, 190 g d'acide néo-décanoïque, 1,011 mole et 358,9 g d'Amsco 140 (un solvant d'éther de pétrole qui est composé à 42% par des hydrocarbures naphténiques et à 58% par des hydrocarbures paraffiniques). On ajoute goutte à goutte 1035 g, 1,006 mole d'hydroxyde d'ammonium en solution aqueuse, à la solution convenablement agitée du mélange d'acide néo-décanoïque de la solution du nitrate céreux et d'éther de pétrole. A la suite de l'achèvement de l'addition de l'hydroxyde d'ammonium, en un laps de deux heures, on ajoute 26,0 g d'une solution aqueuse à 30% de péroxyde d'hydrogène, 0,23 mole à un poids égal d'eau 26 g.

Le mélange réactionnel vire au brun foncé et, après 40 minutes de réaction, on commence à chauffer à 70° pour décomposer les complexes de $Ce^{4+}/H_2O_2$ et l'on continue pendant 20 minutes supplémentaires. Au cours de la décomposition, le mélange réactionnel vire à l'orangé, puis au jaune brillant. Après avoir effectué un refroidissement, on sépare le mélange réactionnel en deux couches, une couche organique supérieure, limpide, orangée et une couche aqueuse inférieure jaune presque limpide.

On sépare les couches et on lave avec 200 g d'eau. On dilue la couche organique avec 300 g d'hexane et l'on évapore l'eau par distillation azéotropique, après quoi l'hexane est distillé.

Le $Ce^{4+}$ présent dans le résidu (581,9 g) est 94% du total du cérium.

Exemple 2

On ajoute dans le même récipient réactionnel que celui décrit à l'exemple 1, 29,4 g d'une solution de nitrate céreux dans l'eau, titrant 23,79% de Ce (0,05 mole), 38,0 g d'acide néo-décanoïque (0,20 mole) et 72,6 g d'Amsco 140. On ajoute goutte à goutte la solution aqueuse d'hydroxyde d'ammonium 167,2 g (0,16 mole) au mélange convenablement agité d'acide néo-décanoïque, de solution aqueuse du nitrate céreux et du solvant d'éther de pétrole, en un laps de 10 minutes. On ajoute ensuite la solution aqueuse de peroxyde d'hydrogène, 5,2 g, $H_2O_2$ à 30% en même temps qu'un poids égal d'eau, 5,2 g. Le mélange vire au brun foncé et la température commence à s'élever. Après environ quarante minutes de réaction, on commence à chauffer à 78° C en un laps de 10 minutes pour décomposer le complexe de $Ce^{4+}/H_2O_2$, après quoi le mélange réactionnel devient jaune orangé puis jaune.

On refroidit le mélange réactionnel à 40° C et l'on sépare le mélange en deux couches, une couche supérieure organique jaune nacré et une couche inférieure aqueuse faiblement jaune.

On élimine la solution d'hexane humide par distillation azéotropique. On filtre la solution refroidie à travers un filtre Supercel à titre d'auxiliaire de filtration. La collecte de néo-décanoate cérique solide, orangé brillant est de 0,26 g. Après distillation de l'hexane de la couche organique, on récupère 119,6 g de produit titrant 5,09% de $Ce^{4+}$.

Exemple 3

Dans un ballon réactionnel on ajoute 176,4 g d'une solution aqueuse de nitrate céreux titrant 23,79% de cérium (0,3 mole), 169,2 g d'acide néo-décanoïque (0,9 mole) et 208,8 g d'Amsco 140, et l'on fait démarrer l'agitation. On ajoute goutte à goutte une solution aqueuse d'hydroxyde d'ammonium, 1044,4 g (1,009 mole) à ce mélange convenablement agité, en un laps de une heure entre 22 et 23° C. A la conclusion de cette période, le pH est de 7. On ajoute alors du peroxyde d'hydrogène (0,2 mole sous la forme d'une solution à 30%) en même temps qu'une quantité égale d'eau, en un laps de quelques minutes. On laisse le mélange réagir pendant 20 Minutes et l'on porte ensuite la température à 70° C puis on l'y tient pendant 20 minutes, pour décomposer le complexe de $Ce^{4+}/H_2O_2$. On refroidit ensuite le mélange réactionnel à 40° C, ce qui entraîne la séparation en deux couches: une couche organique supérieure qui est visqueuse et de couleur brun clair, sous la forme d'une émulsion, et une couche inférieure aqueuse qui est incolore et limpide.

On ajoute au mélange 100 g d'hexane. On sépare ensuite les couches. On effectue l'extraction de la couche aqueuse avec deux portions chacune de 130 g d'hexane et l'on combine ensuite les lavages organiques avec la couche organique, on chasse l'eau par entraînement azéotropique de la couche organique après quoi on élimine l'hexane par distillation. La solution résiduelle dans Amsco, 443,1 g, titre 1,3% de $Ce^{4+}$.

Exemple 4

Dans un ballon de 500 ml, à fond rond, muni d'un dispositif d'agitation rapide et d'un entonnoir d'addition, on charge 29,4 g d'une solution aqueuse de nitrate céreux, titrant 23,86% de Ce (0,05 mole), 29,3 g d'acide 2-éthyl-hexoïque (0,20 mole) et 81 g d'Amsco 140. On ajoute ensuite goutte à goutte une solution aqueuse d'hydroxyde d'ammonium, 205,7 g, 2,6% de $NH_3$, en un laps de 40 minutes. A la fin de l'addition, le pH du mélange réactionnel est de 7,5. La couche organique est légèrement visqueuse et la couche aqueuse est limpide. On ajoute ensuite 5,22 g de peroxyde d'hydrogène à 30% de $H_2O_2$, en agitant vigoureusement. Il se forme presque aussitôt une coloration brun foncé et la couche organique devient moins visqueuse. On continue l'agitation pendant 45 minutes. A l'achèvement de ce délai de réaction, on débute le chauffage jusqu'à 70° C, pour détruire les complexes de $Ce^{4+}/H_2O_2$ qui n'ont pas réagi. Après avoir chauffé jusqu'à 75° C, on entretient cette température pendant trente minutes. La coloration brun foncé disparait ce qui donne lieu à la formation d'une huile orangé-jaune clair et à une couche aqueuse.

On refroidit le mélange réactionnel jusqu'à 20° C avec de la glace. On y ajoute ensuite 5 g d'une solution d'hydroxyde d'ammonium suivis par 1,53 g d'une solution de peroxyde d'hydrogène à 30% et l'on agite ensuite pendant 30 minutes. On chauffe alors le mélange jusqu'à 70° C et on maintient cette température pendant 30 minutes.

On refroidit ensuite le mélange réactionnel à 20° C, température à laquelle le pH est de 7,0. On y ajoute alors 1,53 g de solution de peroxyde d'hydrogène à 30% de $H_2O_2$. On agite le mélange réactionnel pendant une heure et ensuite on fait démarrer le chauffage à 70° C. Le mélange réactionnel est entretenu à la température de 72° C pendant 30 minutes après quoi on refroidit à 20° C dans un bain d'eau et de glace mélangées.

On sépare la couche organique de la couche aqueuse et on la lave avec deux portions d'eau de 25 g chacune. Le poids de la couche organique est de 110,0 g. On ajoute alors 100 g d'hexane et l'on fait démarrer le reflux pour éliminer l'eau. On élimine ensuite l'hexane sous pression de 40 à 80 mm Hg et à une température de 42 à 62° C. Le produit obtenu est une huile vert-jaune, 114,6 g 5,17% de $Ce^{4+}$.

Exemple 5

Dans le système réactionnel selon l'exemple 4, on place 29,4 g d'une solution aqueuse de nitrate céreux titrant 23,86% de cérium (0,05 mole), 50,5 g (0,20 mole) d'acide naphténique et 59,3 g d'Amsco 140. On débute l'agitation et l'on agite alors goutte à goutte la solution aqueuse d'hydroxyde d'ammonium, 170,0 g (0,170 mole) en un laps de 40 minutes. Ensuite, on ajoute 3,1 g de $H_2O_2$ en solution aqueuse de $H_2O_2$ à 30% (0,0327 mole) au mélange vigoureusement agité. Il se forme une coloration brun foncé. On poursuit l'agitation pendant 45 minutes, on chauffe le mélange à 75° C pour détruire les complexes de $Ce^{4+}/H_2O_2$ et on maintient cette température pendant 30 minutes. On refroidit alors le mélange dans un bain d'eau et de glace mélangées jusqu'à 20° C, température à laquelle le mélange se sépare en deux couches. On enlève la couche organique, on y ajoute 100 g d'hexane et l'on porte ensuite le mélange par chauffage jusqu'au reflux. On continue de chauffer au reflux pour entraîner l'eau par azéotropie. On distille par la suite l'hexane qui reste sous basse pression ce qui fournit une matière huileuse que l'on sèche sur du sulfate de sodium. A l'analyse, le total du cérium est de 3,07% dont 70% soit 2,15% est en $Ce^{4+}$. Poids: 214 g.

Exemple 6

| CHARGES | QUANTITE | POIDS MOL. | % | MOLES | RAPPORT MOLAIRE |
|---|---|---|---|---|---|
| $Ce(NO_3)_3$ solution | 29,4 g | 140,12 | 23,86 $Ce^{3+}$ | 0,05 (Ce) | 1,00 |
| Amsco | 61,6 g | | | | 1232 g/ mole Ce |
| Acide naphténique | 48,2 g | 240,8 | | 0,200 | 4,00 |
| NaOH solution | 156 ml | 40 | 0,980 N | 0,153 | 3,06 |
| $H_2O_2$ solution | 4,8 g | 34 | 32,7 | 0,046 | 1,84 |

On munit un ballon à fond rond de 500 ml à trois tubulures, d'un agitateur mécanique, d'un thermomètre, d'une électrode pour la mesure du pH et d'un entonnoir à robinet.

On combine la solution de cérium, l'acide naphténique et l'Amsco 140 et l'on agit vigoureusement pendant que l'on ajoute en un laps de 2 h1/2 une solution de NaOH. On ajoute le peroxyde dans un égal volume d'eau en un laps de 5 minutes, ce qui entraîne la formation d'une coloration rouge foncé. On laisse le mélange sous agitation pendant 20 heures. On chauffe alors ce mélange à 70°C pendant 1/2 heure, on le refroidit puis on titre la couche organique ce qui donne 73% de cérique.

Exemple 7

| CHARGES | QUANTITES | POIDS MOL. | % | RAPPORT MOLAIRE |
|---|---|---|---|---|
| $Ce(NO_3)_3$ solution | 29,4 g | 140,12 | 23,86 Ce | 1 |
| Amsco 140 | 81,0 g | | | |
| Acide octoïque | 29,3 g | 144,2 | 98,2 | 4 |
| NaOH solution | 165,4 ml | 40 | 0,980 N | 3,24 |
| $H_2O_2$ solution | 4,8 g | 34 | 32,9 | 1,84 |

On munit un ballon à trois tubulures, à fond rond, de 500 ml d'un agitateur mécanique, d'un thermomètre, d'une électrode pour la mesure du pH et d'un entonnoir à robinet.

On combine la solution de cérium, l'acide octoïque et l'Amsco et l'on agit vigoureusement pendant que l'on ajoute la solution de NaOH, en un laps de deux heures.

On ajoute la solution de peroxyde d'hydrogène (4,8 g) et l'on agit le mélange pendant dix-sept heures. On chauffe le mélange à 70°C pendant 1/2 heure, on le refroidit et ensuite on effectue un titrage qui donne 70% de produit cérique. Une seconde portion de 3,4 g de peroxyde d'hydrogène est ajoutée et l'on continue d'agiter pendant une nuit et avec un bref chauffage pour donner 91% de produit cérique. Les deux couches se séparent très rapidement et nettement.

Exemple 8

On mélange 2,0 g d'éthyl-hexoate céreux solide avec 50,0 g d'Amsco 140 et l'on agite jusqu'à ce que l'on ait approximativement 80% de dissout. On ajoute la solution de $H_2O_2$ (0,38 g d'une solution aqueuse à 10%) et l'on agite le mélange afin d'obtenir une solution rouge orangé qu'on laisse reposer pendant deux heures et que l'on place ensuite dans un bain à 70% pendant trente minutes pour décomposer les complexes de Ce — $H_2O_2$. La coloration s'éclaircit après quoi on laisse le mélange reposer, refroidir et décanter.

Le titrage avev une solution de sulfate ferro-ammoniacal indique qu'il y a 24% au total de $Ce^{4+}$.

Exemple 9

On ajoute 2,46 g de naphténate céreux solide à 50,0 g d'Amsco 140 et l'on agite ensuite puis on chauffe à 60°C pour obtenir une solution que l'on refroidit à 25°C. On ajoute ensuite une solution de $H_2O_2$ (0,58 g d'une solution aqueuse à 10%) et l'on agit le mélange pendant cinq minutes, on le place dans un bain à 70°C pendant 30 minutes puis on le laisse refroidir jusqu'à 25°C. On prélève un échantillon de la couche supérieure et on effectue un titrage avec du sulfate ferro-ammoniacal ce qui indique une teneur totale de 46,2% en cérium $Ce^4$.

Exemple 10

On ajoute 1,0 g d'acide éthylhexoïque à 2 g d'éthylhexoate céreux solide. Par chauffage et l'addition de 50 g d'Amsco, le tout passe en solution. On ajoute du peroxyde d'hydrogène (solution à 10%) 0,60 g à la température ambiante et après cinq minutes, on chauffe le mélange réactionnel à 70° C puis on le refroidit. On ajoute une seconde portion de 0,6 g de $H_2O_2$ à la température ambiante puis on chauffe à nouveau à 70° C. L'analyse indique que 1,24% du total de Ce est à l'état cérique.

Exemple 11

On place 4,4 g d'acide 2-éthylhexoïque dans un ballon. On y ajoute 4,0 g d'une solution à 30% de NaOH, suivie par 0,57 g d'une solution à 30% de $H_2O_2$, on agite et on ajoute 5,9 g d'une solution de $Ce(NO_3)_3$ ce qui conduit à une solution orangée qui se solidifie après 3 minutes. On ajoute 10,5 g d'Amsco 140 pour faire une solution aqueuse, que l'on agit pendant 5 minutes et qu'on place alors dans un bain à 70° C pendant 30 minutes. On laisse la solution refroidir et l'on prélève des échantillons de la couche organique pour l'analyse par titrage

$$\text{poids/poids \%} \frac{\text{cérique}}{\text{total de Ce}} = 12\%$$

Des additions répétées de $H_2O_2$ augmentent la quantité du produit cérique.

| CHARGES | POIDS | TITRAGE % | POIDS 100% | MOLES |
|---|---|---|---|---|
| 2-éthylhexoate céreux | 2,0 | 24,0 | 0,48 g Ce | 0,0034 |
| Acide 2-éthylhexoïque | 1,0 | 98,3 | 0,9030 | 0,0070 |
| Amsco 140 | 50,0 | | 50,0 | |
| $H_2O_2$ | 1,16 | 10 | 0,1166 | 0,034 |

On combine le 2-éthylhexoate céreux et l'acide 2-éthylhexoïque. On ajoute 50,0 g d'Amsco 140 et 0,58 g de $H_2O_2$ (à 10% poids/poids), on agite pendant 5 minutes et l'on chauffe à 70° C pendant 30 minutes et ensuite on refroidit.

$$\text{poids/poids \%} = \frac{\text{cérique}}{\text{total de Ce}} = 46{,}4\%$$

On ajoute une seconde fois 0,58 g de $H_2O_2$ (à 10% poids/poids) on agite pendant cinq minutes et l'on chauffe à 70° C pendant trente minutes et l'on refroidit ensuite.

$$\text{poids/poids \%} = \frac{\text{cérique}}{\text{total de Ce}} = 67{,}8\%$$

**Revendications**

1. Procédé pour la préparation de carboxylates cériques, caractérisé en ce que, dans une première étape, on opère l'oxydation d'un carboxylate céreux avec du péroxyde d'hydrogène, dans un système en deux phases qui est constitué, d'une part, par une phase aqueuse de peroxyde d'hydrogène, dont la valeur du pH est de 6 au moins et, d'autre part, par une phase organique constituée par une solution de carboxylate céreux dans un solvant hydrocarboné, l'oxydation transforme l'ion céreux en ion cérique et conduit à la formation d'une solution de carboxylate cérique dans le solvant hydrocarboné; dans une deuxième étape, on chauffe le mélange réactionnel à une température à laquelle se décomposent d'éventuels complexes cériques du peroxyde d'hydrogène présents et, dans une troisième étape, on sépare la phase organique contenant le carboxylate cérique d'avec la phase aqueuse du mélange réactionnel.

2. Procédé selon la revendication 1, caractérisé par le fait que l'on utilise le carboxylate céreux directement à titre de matière de départ, en solution dans un solvant hydrocarboné qui n'est pas miscible avec l'eau et la solution aqueuse de peroxyde d'hydrogène combinée avec cette solution pour former le système réactionnel en deux phases.

3. Procédé selon la revendication 1, caractérisé par le fait que l'on prépare le carboxylate céreux à partir d'un sel céreux inorganique et d'un acide carboxylique par l'addition d'une base, et que l'on utilise directement le mélange réactionnel dans l'oxydation par le peroxyde d'hydrogène, sans séparation du carboxylate céreux.

4. Procédé selon la revendication 3, caractérisé par le fait que l'on ajoute un hydroxyde, un carbonate ou un bicarbonate d'un métal alcalin à une solution aqueuse bien agitée d'un sel céreux inorganique, soluble dans l'eau et l'acide carboxylique correspondant au moins selon une quantité stoechiométrique et que l'on effectue l'extraction du carboxylate céreux du mélange réactionnel aqueux avec un solvant hydrocarboné qui n'est pas miscible avec l'eau et dans lequel il est soluble.

5. Procédé selon la revendication 4, caractérisé par le fait que le solvant est présent au moment de l'addition de la base, ce qui forme ainsi un système à deux phases.

6. Procédé selon la revendication 4, caractérisé par le fait que l'on ajoute le solvant à la fin de la précipitation du carboxylate céreux dans la phase aqueuse.

7. Procédé selon la revendication 4, caractérisé par le fait que l'on ajoute le peroxyde d'hydrogène sous la forme d'une solution aqueuse peu après que l'on a achevé l'addition de la base et avant la séparation des deux couches et que le pH du mélange réactionnel avant l'addition de $H_2O_2$ est supérieur à 6.

8. Procédé selon la revendication 7 caractérisé par le fait que l'on effectue l'addition de $H_2O_2$ après avoir-ajouté la base, en une proportion stoechiométrique, en fonction de la quantité d'acide présent au total.

9. Procédé selon la revendication 7, caractérisé par le fait que l'on n'effectue l'addition de $H_2O_2$ qu'après avoir ajouté au moins la presque totalité de la quantité stoechiométrique de la base correspondant à la quantité de cérium et que l'on ajuste la valeur du pH du mélange réactionnel au-dessus de 6.

10. Procédé selon la revendication 1, caractérisé par le fait que l'on choisit la quantité de peroxyde d'hydrogène en fonction de l'importance désirée de la transformation de l'état céreux en l'état cérique dans la gamme comprise entre environ 5% minimum et environ 95%.

11. Procédé selon la revendication 10 caractérisé par le fait que l'on effectue au moins deux additions par portions de peroxyde d'hydrogène, chaque portion ajoutée étant suivie par un chauffage et un refroidissement, pour décomposer les complexes cériques $-H_2O_2$.

12. Procédé selon la revendication 1, caractérisé par le fait que l'on prépare le carboxylate cérique par l'addition d'une solution aqueuse de peroxyde d'hydrogène à une solution aqueuse de carboxylate d'un métal alcalin ou d'ammonium, suivi par l'addition d'une solution aqueuse de sel céreux, ce qui forme le carboxylate cérique.

13. Procédé selon la revendication 12, caractérisé par le fait que l'on ajoute une solution aqueuse de peroxyde d'hydrogène à la solution aqueuse de sel céreux avant le mélange avec la solution de carboxylate alcalin ou d'ammonium.

14. Procédé selon la revendication 12, caractérisé par le fait que l'on ajoute la solution aqueuse de peroxyde d'hydrogène à une vitesse contrôlée au cours du mélange avec la solution du carboxylate alcalin.

15. Procédé selon la revendication 1, caractérisé par le fait qu'on le conduit à une température qui est comprise dans la gamme allant de la température ambiante à 70° C.

16. Procédé selon la revendication 1, caractérisé par le fait que l'on choisit le carboxylat céreux parmi le groupe de composés comprenant le naphténate céreux, le 2-éthylhexanoate céreux et le néo-décanoate céreux.

17. Procédé selon la revendication 1, caractérisé par le fait que le carboxylate provient d'un acide carboxylique choisi parmi le groupe de composés comprenant des acides carboxyliques aliphatiques et cycloaliphatiques, saturés et non saturés et leurs mélanges, qui présentent entre sept et dix-huit atomes de carbone.

18. Procédé selon la revendication 1, caractérisé par le fait que l'on choisit le solvant hydrocarboné liquide, non miscible avec l'eau parmi le groupe constitué par des solvants hydrocarbonés aliphatiques, cyclo-aliphatiques et aromatiques présentant entre six à quatorze atomes de carbone.

19. Procédé selon la revendication 1, caractérisé en ce qu'on le conduit pour avoir au moins 5% de transformation de l'état céreux en l'état cérique.

20. Procédé selon la revendication 1, caratérisé en ce qu'on le conduit pour avoir une transformation de l'état céreux en l'état cérique comprise dans la gamme de 30 à 60%.

21. Procédé selon la revendication 1, caractérisé en ce qu'on le conduit pour avoir une transformation de l'état céreux en l'état cérique compris dans la gamme de 50 à 95%.

**Patentansprüche**

1. Verfahren zur Herstellung von Cer(IV)-carboxylaten, dadurch gekennzeichnet, daß man in einer ersten Stufe ein Cer(III)-carboxylat mit Wasserstoffperoxid in einem Zweiphasensystem oxydiert, das sich zum einen Teil aus einer wäßrigen Wasserstoffperoxidphase mit einem pH-Wert von mindestens 6, und zum anderen Teil aus einer organischen Phase zusammensetzt, die aus einer Cer(III)-carboxylatlösung in einem Kohlenwasserstoff-Lösungsmittel besteht, wobei das Cer(III)- zum Cer(IV)-Ion oxydiert und eine Cer(IV)-carboxylatlösung in dem Kohlenwasserstoff-Lösungsmittel gebildet wird; in einer zweiten Reaktionsstufe erwärmt man das Reaktionsgemisch auf eine Temperatur, bei der sich mögliche Cer(IV)/Wasserstoffperoxid-Komplexe zersetzen, und in einer dritten Stufe trennt man die organische Phase, die das Cer(IV)-carboxylat enthält, von der wäßrigen Phase des Reaktionsgemischs ab.

2. Verfahren nach Anspruch 1, dadurch gekennzeichnet, daß man das Cer(III)-carboxylat, gelöst in einem mit Wasser nicht mischbaren Kohlenwasserstoff, direkt als Ausgangsmaterial zusammen mit der wäßrigen Lösung des Wasserstoffperoxids als Zweiphasenreaktionsgemisch einsetzt.

3. Verfahren nach Anspruch 1, dadurch gekennzeichnet, daß man das Cer(III)-carboxylat ausgehend von einem anorganischen Cer(III)-Salz und einer Carbonsäure durch Zugabe einer Base herstellt, und daß man das Reaktionsgemisch direkt mit Wasserstoffperoxid oxydiert ohne das Cer(III)-carboxylat abzutrennen.

4. Verfahren nach Anspruch 3, dadurch gekennzeichnet, daß man ein Hydroxid, ein Carbonat oder ein Bicarbonat eines Alkalimetalls zu einer gut gerührten Lösung eines wasserlöslichen anorganischen Cer(III)-Salzes und mindestens die stöchiometrische Menge der entsprechenden Carbonsäure zugibt und daß man das Cer(III)-carboxylat mit einem Kohlenwasserstoff-Lösungsmittel, das mit Wasser nicht mischbar ist und das Cersalz löst, aus dem Reaktionsgemisch extrahiert.

5. Verfahren nach Anspruch 4, dadurch gekennzeichnet, daß das Lösungsmittel im Augenblick der Zugabe der Base anwesend ist, wodurch ein Zweiphasensystem gebildet wird.

6. Verfahren nach Anspruch 4, dadurch gekennzeichnet, daß man das Lösungsmittel zu der wäßrigen Phase zusetzt, wenn die Fällung des Cer(III)-carboxylats beendet ist.

7. Verfahren nach Anspruch 4, dadurch gekennzeichnet, daß man das Wasserstoffperoxid in Form einer wäßrigen Lösung zusetzt, kurz nachdem die Zugabe der Base beendet ist und bevor sich die zwei Schichten getrennt haben, und dadurch, daß der pH-Wert des Reaktionsgemischs vor der Zugabe des $H_2O_2$ über 6 beträgt.

8. Verfahren nach Anspruch 7, dadurch gekennzeichnet, daß man die Zugabe von $H_2O_2$ nach der Zugabe der Base in stöchiometrischer Menge in Abhängigkeit von der vorliegenden Gesamtsäuremenge durchführt.

9. Verfahren nach Anspruch 7, dadurch gekennzeichnet, daß man das $H_2O_2$ erst nach der Zugabe von mindestens nahezu der gesamten stöchiometrischen Menge der Base, die der Cermenge entspricht, zugibt, und daß man den pH-Wert des Reaktionsgemischs oberhalb 6 einstellt.

10. Verfahren nach Anspruch 1, dadurch gekennzeichnet, daß man die Menge des Wasserstoffperoxids entsprechend der gewünschten Umwandlung von Cer(III) in Cer(IV) im Bereich zwischen mindestens etwa 5 und etwa 95% wählt.

11. Verfahren nach Anspruch 10, dadurch gekennzeichnet, daß man das Wasserstoffperoxid in wenigstens zwei Portionen zugibt, wobei nach jeder Zugabe eine Erwärmung und eine Abkühlung erfolgt zur Zersetzung der Cer(IV)/$H_2O_2$-Komplexe.

12. Verfahren nach Anspruch 1, dadurch gekennzeichnet, daß man das Cer(IV)-carboxylat durch Zugabe einer wäßrigen Wasserstoffperoxidlösung zu einer wäßrigen Lösung eines Alkalimetall- oder Ammoniumcarboxylats, gefolgt von der Zugabe einer wäßrigen Lösung von Cer(III)-Salz, was zur Bildung des Cer(IV)-carboxylats führt, herstellt.

13. Verfahren nach Anspruch 12, dadurch gekennzeichnet, daß man eine wäßrige Lösung von Wasserstoffperoxid zu einer wäßrigen Lösung eines Cer(III)-Salzes vor der Mischung mit der Lösung des Alkali- oder Ammoniumcarboxylats zugibt.

14. Verfahren nach Anspruch 12, dadurch gekennzeichnet, daß man die wäßrige Lösung von Wasserstoffperoxid mit kontrollierter Geschwindigkeit während der Vermischung mit der Lösung des Alkalicarboxylats zugibt.

15. Verfahren nach Anspruch 1, dadurch gekennzeichnet, daß man es bei einer Temperatur zwischen Umgebungstemperatur und 70° C durchführt.

16. Verfahren nach Anspruch 1, dadurch gekennzeichnet, daß man das Cer(III)-carboxylat aus der Gruppe der Verbindungen auswählt, die das Cer(III)-naphtenat, -2-ethylhexanoat und -neo-decanoat umfaßt.

17. Verfahren nach Anspruch 1, dadurch gekennzeichnet, daß das Carboxylat sich von einer Carboxylatsäure ableitet, die aus der Gruppe der gesättigten und nicht gesättigten aliphatischen und cycloaliphatischen Carboxylsäuren mit 7 bis 18 Kohlenstoffatomen und ihrer Mischungen stammt.

18. Verfahren nach Anspruch 1, dadurch gekennzeichnet, daß man das flüssige Kohlenwasserstoff-Lösungsmittel, das mit Wasser nicht mischbar ist, aus der Gruppe der aliphatischen, cycloaliphatischen und aromatischen Kohlenwasserstoff-Lösungsmittel mit 6 bis 14 Kohlenstoffatomen auswählt.

19. Verfahren nach Anspruch 1, dadurch gekennzeichnet, daß man es so führt, daß man mindestens 5% Umwandlung von Cer(III) in Cer(IV) erhält.

20. Verfahren nach Anspruch 1, dadurch gekennzeichnet, daß man es so führt, daß man eine Umwandlung von Cer(III) in Cer(IV) im Bereich von 30 bis 60% erhält.

21. Verfahren nach Anspruch 1, dadurch gekennzeichnet, daß man es so führt, daß man eine Umwandlung von Cer(III) in Cer(IV) im Bereich von 50 bis 95% erhält.

**Claims**

1. Process for the preparation of ceric carboxylates, characterised in that, in a first step, oxidation of a cerous carboxylate is carried out with hydrogen peroxide, in a two-phase system consisting, on the one hand, of an aqueous hydrogen peroxide phase, whose pH value is at least 6 and, on the other hand, an organic phase consisting of a solution of cerous carboxylate in a hydrocarbon solvent, the oxidation converts the cerous ion to ceric ion and results in the formation of a solution of ceric carboxylate in the hydrocarbon solvent; in a second step the reaction mixture is heated to a temperature at which ceric hydrogen peroxide complexes which may be present are decomposed and, in a third step, the organic phase containing the ceric carboxylate is separated from the aqueous phase of the reaction mixture.

2. Process according to Claim 1, characterised in the use of the cerous carboxylate directly as starting material, in solution in a water-immiscible hydrocarbon solvent, and the aqueous hydrogen peroxide solution combined with this solution to form the two-phase reaction system.

3. Process according to Claim 1, characterised in that the cerous carboxylate is prepared from an inorganic cerous salt and a carboxylic acid by the addition of a base, and that the reaction mixture is used directly in the oxidation by hydrogen peroxide, without isolation of the cerous carboxylate.

4. Process according to Claim 3, characterised in that an alkali metal hydroxide, carbonate or bicarbonate is added to a well-stirred aqueous solution of a water-soluble inorganic cerous salt and the corresponding carboxylic acid in at least a stoichiometric quantity, and that extraction of the cerous carboxylate from the aqueous reaction mixture is carried out with a water-immiscible hydrocarbon solvent in which it is soluble.

5. Process according to Claim 4, characterised in that the solvent is present at the time of addition of the base, thus forming a two-phase system.

6. Process according to Claim 4, characterised in that the solvent is added at the end of the precipitation of cerous carboxylate in the aqueous phase.

7. Process according to Claim 4, characterised in that the hydrogen peroxide is added in the form of an aqueous solution shortly after the addition of the base has been completed and before separation of the two layers and in that the pH of the reaction mixture is higher than 6 before the addition of $H_2O_2$.

8. Process according to Claim 7, characterised in that the addition of $H_2O_2$ is carried out after the base has been added, in a stoichiometric proportion, depending on the total quantity of acid present.

9. Process according to Claim 7, characterised in that the addition of $H_2O_2$ is carried out only after at least almost all of the stoichiometric quantity of the base corresponding to the quantity of cerium has been added, and that the pH value of the reaction mixture is adjusted above 6.

10. Process according to Claim 1, characterised in that the quantity of hydrogen peroxide is chosen as a function of the required degree of conversion from the cerous state to the ceric state in the range of between approximately at least 5% and approximately 95%.

11. Process according to Claim 10, characterised in that at least two portionwise additions of hydrogen peroxide are carried out, each added portion being followed by heating and cooling, to decompose the ceric-$H_2O_2$ complexes.

12. Process according to Claim 1, characterised in that the ceric carboxylate is prepared b the addition of an aqueous hydrogen peroxide solution to an aqueous solution of an alkali metal carboxylate or ammonium carboxylate, followed by the addition of an aqueous solution of cerous salt, which produces the ceric carboxylate.

13. Process according to Claim 12, characterised in that an aqueous hydrogen peroxide solution is added to the aqueous solution of cerous salt before mixing with the solution of alkali metal carboxylate or ammonium carboxylate.

14. Process according to Claim 12, characterised in that the aqueous hydrogen peroxide solution is added at a controlled rate during the mixing with the alkali metal carboxylate solution.

15. Process according to Claim 1, characterised in that it is carried out at a temperature in the range from ambient temperature to 70° C.

16. Process according to Claim 1, characterised in that the cerous carboxylate is chosen from the group of compounds comprising cerous naphthenate, cerous 2-ethylhexanoate and cerous neodecanoate.

17. Process according to Claim 1, characterised in that the carboxylate originates from a carboxylic acid chosen from the group of compounds comprising saturated and unsaturated aliphatic and alicyclic carboxylic acids and their mixtures, containing from seven to eighteen carbon atoms.

18.Process according to Claim 1, characterised in that the liquid, water-immiscible hydrocarbon solvent is chosen from the group consisting of aliphatic, alicyclic and aromatic hydrocarbon solvents containing from six to fourteen carbon atoms.

19. Process according to Claim 1, characterised in that it is carried out to produce at least 5% conversion from the cerous state to the ceric state.

20. Process according to Claim 1, characterised in that it is carried out to produce a conversion in the range from 30 to 60% from the cerous state to the ceric state.

21. Process according to Claim 1, characterised in that it is carried out to produce a conversion from the cerous state to the ceric state in the range from 50 to 95%.